(19) Europäisches Patentamt
European Patent Office
Office européen des brevets

(11) **EP 4 621 039 A1**

(12) **EUROPEAN PATENT APPLICATION**

(43) Date of publication:
24.09.2025 Bulletin 2025/39

(21) Application number: 24164663.7

(22) Date of filing: 19.03.2024

(51) International Patent Classification (IPC):
*C12M 1/00* (2006.01)    *C12M 1/34* (2006.01)

(52) Cooperative Patent Classification (CPC):
C12M 29/22; C12M 29/20; C12M 29/24;
C12M 41/34; C12M 41/40

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH MA MD TN

(71) Applicant: COLIPI GmbH
21079 Hamburg (DE)
(72) Inventors:
• UTESCH, Tyll
  21423 Winsen (Luhe) (DE)
• ARBTER, Philipp
  21109 Hamburg (DE)
• HEUER, Jonas
  22765 Hamburg (DE)
• WEBERS, Maximilian
  22078 Hamburg (DE)

(74) Representative: Eisenführ Speiser
Patentanwälte Rechtsanwälte PartGmbB
Gollierstraße 4
80339 München (DE)

(54) **PRESSURE CONTROLLED GAS FERMENTATION**

(57)    The present invention relates to a process for controlling gas fermentation and to an apparatus for cultivation of microorganisms which is useful for carrying out said process.

FIG. 1

## Description

### Technical field

The present invention relates to a process for controlling gas fermentation and to an apparatus for cultivation of microorganisms which is useful for carrying out said process.

### Background of the Invention

Biotechnological production of bioproducts, for example biobased chemicals, is long known and promises the provision of chemicals for commercial use, e.g. in the chemical industry, in a more sustainable and environmentally friendly way. As an example, so-called biofuels like bioethanol or biodiesel have been produced with bioprocesses in order to replace fossil fuels derived from oil (see, e.g., WO 2011/088364 A9, WO 2009/133351 A2). However, considering global warming due to increasing amounts of $CO_2$ in the atmosphere, also bioprocesses for producing chemicals in a large scale will have to minimize release of $CO_2$ in order to be sustainable.

One way to reduce $CO_2$ emissions in bioprocesses is to (re-)use $CO_2$ produced in the ongoing process or in an upstream process. WO 2011/088364 A9, WO 2009/133351 A2, and WO 2011/139804 A2 describe the fixation of $CO_2$ produced in a first (bio) process in a second bioprocess in order to re-use produced $CO_2$. WO 2011/088364 A9 discloses a method for converting a carbon source into a lipid, wherein the lipid production is carried out in an aerobic fermenter using the oleaginous yeast *Yarrowia lipolytica*, and carbon dioxide generated during lipid production is converted into a carbon substrate by $CO_2$ fixation carried out by bacteria of the genus Clostridium sp. in an anaerobic fermenter. WO 2009/133351 A2 discloses the production of biofuel using photosynthetic plant cells that fix $CO_2$ produced, for example, by yeast cells producing biofuel. WO 2011/139804 A2 describes, *inter alia*, a method for the capture and fixation of carbon dioxide, e.g. from flue gas, by oxyhydrogen ("Knallgas") bacteria, wherein oxygen and hydrogen can, for example, be provided by electrolysis of water. Biotechnological production of biofuels using "Knallgas" bacteria is also described in Brigham, C. (2019), Perspectives for the biotechnological production of biofuels from CO2 and H2 using Ralstonia eutropha and other 'Knallgas' bacteria, Applied Microbiology and Biotechnology 103, 10.1007/s00253-019-09636-y.

Oxyhydrogen bacteria, which are chemolithotrophic bacteria, require an atmosphere of $H_2$ and $O_2$ for the utilization of $CO_2$ or CO. Gas mixtures of $O_2$ and $H_2$ have the major disadvantage that they become explosive from a fraction of 4 vol% $H_2$ (lower explosion limit, LEL) or 5 vol% $O_2$, with corresponding fractions of the respective other gas. On the other hand, gas mixtures with a fraction of $H_2$ above 75 vol% (upper explosion limit, OEG) allows a fraction of $O_2$ above 5 vol% without the building of an explosive atmosphere.

Besides mitigating the explosion hazard, another major challenge with the known bioprocesses involving oxyhydrogen bacteria is the gas management, in particular controlling and maintaining the desired gas composition in the liquid phase. Solubilities of gases in water as per Henry's Law decrease in the order $CO_2>O_2>H_2$, with 25- and 42-fold solubility of $CO_2$ to $O_2$ and $H_2$ (R. Sander. Compilation of Henry's law constants (version 4.0) for. Atmos. Chem. phys. 2015, 4399-4981; $CO_2=3.3\times10^{-2}$ mol L$^{-1}$ atm$^{-1}$, $O_2=1.3\times10^{-3}$ mol L$^{-1}$ atm$^{-1}$, $H_2=7.8\times10^{-4}$ mol L$^{-1}$ atm$^{-1}$). Thus, as $H_2$ has a poor solubility in comparison to the other gases, introduction and solubility of $H_2$ in the liquid phase proves to be particularly challenging. Simple gassing of $H_2$, and the other gases for that matter, leads to a waste of the gas as gases not introduced and solved into the liquid escape from the system. Due to the costly $H_2$ generation by an electrolyzer, the total cost of fermentation therefore increases significantly. Another challenge is to balance and maintain the fractions of $CO_2$ and $O_2$. When $CO_2$ is supplied via COz-containing substrate gas, e.g. flue gas, the substrate gas may have varying contents of $CO_2$ and other gases depending on the source or emitter, which makes it difficult to maintain the target $CO_2$ content while preventing the accumulation of other gases like $N_2$ or $CH_4$. Poor gas management may lead to an increase in $CO_2$ concentration, resulting in increased carbonation, which in turn hinders the control of the bioprocess in terms of pH. On the other hand, if the $O_2$ fraction rises above 20 vol%, the growth of many $H_2$-oxidizing microorganisms is inhibited.

Due to complex gas management, in which it is crucial to find a balance between the explosion issue on the one hand and an optimal product and biomass yield on the other, the process planning and management of fermentation with oxyhydrogen bacteria presents particular difficulties.

In view of the challenges, therefore, the object of the present invention is to provide a safe and efficient process for controlling gas fermentation in which gas loss is reduced and fluctuations in gas concentration are compensated and balanced. A further object of the invention is to provide an apparatus for the cultivation of microorganisms suitable for the process according to the invention.

### Summary of the Invention

These objects have been solved by the aspects of the present invention as specified hereinafter.

According to a first aspect of the invention, a process for controlling gas fermentation is provided, wherein the process comprises a bioprocess comprising cultivation of a microorganism in a liquid medium as a liquid culture in a bioreactor; wherein during the bioprocess (i) gases comprising molecular $H_2$ and $CO_2$ are introduced into the bioreactor and at least partially dissolved into the liquid culture; (ii) the partial pressure of each of the gases in a gaseous phase in a headspace above the medium is measured; and (iii) the volumetric flow rate of each of the introduced gases is continuously controlled such that the measured partial pressure of each of the gases in the bioreactor is controlled to a corresponding predetermined set partial pressure.

According to an embodiment of the first aspect of the invention, the microorganism is a bacterium, preferably a chemolithotrophic bacterium, more preferably a chemolithoautotrophic bacterium, even more preferably an oxyhydrogen bacterium, particularly preferably from the family *Hydrogenophilaceae,* particularly preferably of the species *Hydrogenophilus thermoluteolus.*

According to another embodiment of the first aspect of the invention, at least 3 different gases, at least 4 different gases, or at least 5 different gases are introduced into the bioreactor during the bioprocess.

According to yet another embodiment of the first aspect of the invention, additionally molecular $O_2$, molecular $N_2$, $CH_4$, and/or CO are introduced into the bioreactor during the bioprocess.

According to yet another embodiment of the first aspect of the invention, any of the introduced gases is introduced separately or at least two or more thereof are introduced together in form of a gas mixture, preferably wherein the gas mixture comprises $CO_2$, $O_2$, $N_2$, and/or $CH_4$.

According to yet another embodiment of the first aspect of the invention, the total pressure during the bioprocess in the bioreactor is controlled to a predetermined set total pressure equal to or above atmospheric pressure, preferably above atmospheric pressure.

According to a particular embodiment of the first aspect of the invention, the set total pressure is between at least 1 bar to at most 10 bar, preferably between at least 1.5 bar to at most 6 bar, more preferably between 2 bar and 4 bar, particularly preferably at about 3 bar.

According to another embodiment of the first aspect of the invention, the partial pressure of any of the gases in the gas atmosphere is controlled by a separate controller, preferably a PI controller configured for controlling the volumetric flow rate of the corresponding introduced gas or a gas mixture comprising said corresponding introduced gas.

According to yet another embodiment of the first aspect of the invention, the bioprocess is performed at a temperature between 20 to 60 °C, preferably between 30 to 58 °C, more preferably between 40 to 56 °C, particularly preferably between 45 to 54 °C, particularly preferably between 50 to 52 °C.

According to yet another embodiment of the first aspect of the invention, wherein at most 0.1 vol% of the $H_2$, $CO_2$, and/or molecular $O_2$ present in the bioreactor is removed from the bioreactor during the bioprocess per hour, or wherein no $H_2$, $CO_2$, and/or molecular $O_2$ present in the bioreactor is removed from the bioreactor during the bioprocess.

According to yet another embodiment of the first aspect of the invention, excess $N_2$ and/or excess $CH_4$ is removed in a controlled manner from the bioreactor, preferably by membrane-based separation of $N_2$ and/or $CH_4$.

According to yet another embodiment of the first aspect of the invention, the gas atmosphere in the bioreactor comprises 21 vol% or less of $O_2$, 15 vol% or less of $CO_2$, and (a) more than 75 vol% of $H_2$, in cases wherein the gas atmosphere contains no $N_2$, preferably about 76 vol% of $H_2$, about 14 vol% of $O_2$, and about 10 vol% of $CO_2$; or (b) less than 4 vol% of $H_2$, in cases wherein the gas atmosphere contains $N_2$.

According to yet another embodiment of the first aspect of the invention, the medium is agitated during the bioprocess by circulating gas from the gaseous phase in a headspace above the medium into the liquid phase (gas cycling), by introducing gas from external sources into the liquid phase, by mixer agitation, and/or by circulating and spraying medium from the liquid phase into the headspace (liquid cycling).

According to yet another embodiment of the first aspect of the invention, the process is for controlling growth rate and/or production rate of the cultured microorganism.

According to a second aspect of the invention, an apparatus for cultivation of microorganisms is provided, comprising a bioreactor configured for carrying out a bioprocess, wherein the bioprocess comprises the cultivation of microorganisms, preferably chemolithoautotrophic bacteria, in a liquid medium at a total pressure, preferably above atmospheric pressure, and wherein the bioprocess is a gas-consuming process producing biomass, preferably wherein the consumed gases comprise molecular hydrogen $H_2$, $CO_2$ and molecular oxygen $O_2$; a control unit comprising monitoring and analysis means configured for measuring and analyzing process parameters during the bioprocess and a plurality of PI controllers, preferably PID controllers, particularly preferably mass flow controllers driven by a PID logic, wherein each controller is configured for controlling a volumetric flow rate of a gas introduced into the bioreactor based on data of the gas analytic means as outlined by the process of the preceding claims; first gas separation means, preferably a separation membrane, configured for removing $N_2$ and/or $CH_4$ in a controlled manner from the bioreactor; a first fluid connection fluidically connecting an outlet of a first external gas source to a first inlet of the bioreactor and configured for the transfer of a first gas, preferably $H_2$, from the first external gas source into the bioreactor; a second fluid connection fluidically connecting an outlet of a second external gas source to a second inlet of the bioreactor and configured for the transfer of a second gas, preferably $CO_2$, from the second external gas source into the bioreactor; and a third fluid connection fluidically connecting an outlet of a third external gas source to a third inlet of the bioreactor, configured for the transfer of a third gas, preferably $O_2$ and/or a gas mixture comprising $CO_2$, $O_2$, $N_2$, and/or $CH_4$, from the third external gas source into the bioreactor.

According to an embodiment of the second aspect of the invention, the apparatus further comprises an electrolyzer configured for the electrolysis of water into $O_2$ and $H_2$ and comprising a separator configured for separating $O_2$ and $H_2$, wherein the $H_2$ side of the separator is the first external gas source and the $O_2$ side of the separator is the third external gas source; a fourth fluid connection fluidically connecting a first outlet of the bioreactor located at liquid culture level to a fourth inlet of the bioreactor located at headspace level and configured for circulation of liquid medium to the headspace, preferably wherein the fourth inlet comprises spraying means configured for spraying the circulated medium in the headspace; a fifth fluid connection fluidically connecting a second outlet of the bioreactor located at headspace level to a fifth inlet of the bioreactor located at liquid culture level and configured for circulation of the gas from the headspace into the medium, preferably wherein the fifth inlet comprise sparger means configured for dispersing the circulated gas in the medium; a sixth fluid connection fluidically connected to the fifth fluid connection and comprising first gas separation means; and/or a second gas separation means, preferably a gas separation membrane, provided in the third fluid connection and configured for the removal of $N_2$ and/or $CH_4$ in the second gas transferred through the third fluid connection into the bioreactor.

According to another embodiment of the second aspect of the invention, the first inlet, second inlet, and third inlet are located at liquid culture level, preferably at the bottom of the bioreactor, and comprise sparger means configured for dispersing gas in the medium.

## Description of Figures

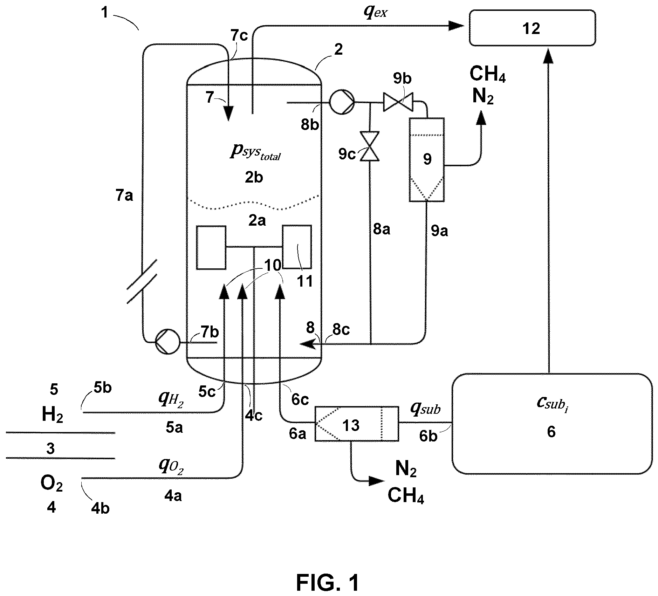

Figure 1 shows a diagram of the pressurized gas fermentation system according to an embodiment of the invention comprising both the first and second separation means 9, 13.

## Detailed Description of the Invention

In order that the present description can be more readily understood, certain terms are first defined. Additional definitions are set forth throughout the detailed description.

It is to be noted that the term "a" or "an" entity refers to one or more of that entity; for example, "a microorganism," is understood to represent one or more microorganisms. As such, the terms "a" (or "an"), "one or more," and "at least one" can be used interchangeably herein.

Furthermore, "and/or" where used herein is to be taken as specific disclosure of each of the two specified features or components with or without the other. Thus, the term "and/or" as used in a phrase such as "A and/or B" herein is intended to include "A and B," "A or B," "A" (alone), and "B" (alone). Likewise, the term "and/or" as used in a phrase such as "A, B, and/or C" is intended to encompass each of the following aspects: A, B, and C; A, B, or C; A or C; A or B; B or C; A and C; A and B; B and C; A (alone); B (alone); and C (alone).

It is understood that wherever aspects are described herein with the language "comprising," otherwise analogous aspects described in terms of "consisting of" and/or "consisting essentially of" are also provided.

Unless defined otherwise, all technical and scientific terms used herein have the same meaning as commonly understood by one of ordinary skill in the art to which this disclosure is related. For example, the Concise Dictionary of Biomedicine and Molecular Biology, Juo, Pei-Show, 2nd ed., 2002, CRC Press; The Dictionary of Cell and Molecular Biology, 3rd ed., 1999, Academic Press; and the Oxford Dictionary Of Biochemistry And Molecular Biology, Revised, 2000, Oxford University Press, provide one of skill with a general dictionary of many of the terms used in this disclosure.

Units, prefixes, and symbols are denoted in their Système International d'Unités (SI) accepted form. Numeric ranges are inclusive of the numbers defining the range. The headings provided herein are not limitations of the various aspects of the disclosure, which can be had by reference to the specification as a whole. Accordingly, the terms defined immediately below are more fully defined by reference to the specification in its entirety.

The term "about" is used herein to mean approximately, roughly, around, or in the regions of. When the term "about" is used in conjunction with a numerical range, it modifies that range by extending the boundaries above and below the numerical values set forth. In general, the term "about" can modify a numerical value above and below the stated value by a variance of, e.g., 10 percent, up or down (higher or lower).

The term "bioproduct" refers to a product of a bioprocess, for example, a chemical compound, in particular organic chemical compound, which is a constituent of, produced by or involves chemical reactions in a living cell, in contrast to, for example, chemicals based on fossil feed stocks. Derived from biomass, biobased chemicals can be structurally identical to existing chemicals derived from fossil sources, or can structurally be different from such chemicals. Examples of biobased chemicals include, for example, fatty acids and fatty acid derivatives, e.g. lipids, alcohols, e.g. diols, and acids, biopolymers or monomers for polymerization, peptides etc. The term also encompasses biomass as a product of a bioprocess, for example living, freeze-dried or hydrolyzed microorganisms. As defined above, the term "biobased chemical" relates to a chemical compound, in particular organic chemical compound, which is a constituent of, produced by or involves chemical reactions in a living cell. The term "biochemical" may also be used synonymously for "biobased chemical". It should be noted that, although the invention may be mainly described in relation to the production of a biobased chemical, and is also mainly intended for this, this should not be construed as excluding the production of biomass, for example, as a bioproduct.

The term "lipid" relates to organic compounds that are soluble in nonpolar organic solvents (like chloroform or ether) and usually insoluble in polar solvents like water, and includes, for example, fats, oils, waxes, phospholipids, and steroids.

The term "bioprocess" used herein relates to any biological process, i.e. a process involving living cells, in particular living microorganisms like bacteria, or functional components of living cells, for example enzymes, cell-free systems or organelles, to obtain a desired product. The product may be a chemical compound, for example organic chemical compound, a mixture of chemical compounds, for example a mixture of organic chemical compounds, or biomass. Examples are the production of lipids from a substrate like glucose, starch, glycerol, or the like.

A "bioreactor" used herein refers to a reactor that comprises a working volume in which a suitable medium, for example an aqueous medium suitable for supporting growth of a culture of microorganisms, e.g. bacterial cells, can be provided and in which biological and/or biochemical processes and reactions can be carried out, or in which conditions can be created under which biological and/or biochemical processes and reactions take place. The processes and reactions can be material transformations, for example the synthesis, modification or degradation of substances by living cells or a cell-free system, or the growth of biomass, i.e. the proliferation of living cells, for example bacterial cells. Depending on the type of process to be carried out and/or the type of microorganisms used to carry out the process, a bioreactor may, for example, be equipped with facilities for aeration/ventilation and/or agitation of the medium.

The term "closed" in relation to the bioreactor or to the vessel means that the photobioreactor or the vessel are closed to the surrounding environment such that the photobioreactor or the vessel can be pressurized. This does not rule out the possibility that the bioreactor or the vessel has openings, connections or the like, via which, for example, a fluid, e.g. a gas, e.g. $CO_2$, or a liquid, can be introduced into or removed from the container.

The terms "Knallgas bacteria", "hydrogen oxidizing bacteria", "$H_2$ oxidizing bacteria", or "oxyhydrogen bacteria" are used herein interchangeably and relate to a physiologically defined group of bacteria that is able to grow autotrophically, i.e. to fix carbon dioxide, while oxidizing hydrogen ($H_2$) and using oxygen ($O_2$) as terminal electron acceptor. The abbreviation HOB may be used for the terms hydrogen or Hz oxidizing bacteria. The term "oxyhydrogen" is used herein synonymously for "Knallgas". "Knallgas" is a mixture of gaseous hydrogen and gaseous oxygen. Knallgas bacteria are aerobic facultatively chemolithoautotrophic bacteria. Examples of Knallgas bacteria are *Hydrogenophilus thermoluteolus*, *Hydrogenobacter thermophilus, Hydrogenovibrio marinus, Cupriavidus metallidurans, Rhodococcus opacus, Xantobacter autotrophicus*, and *Cupriavidus necator* (formerly *Alcaligenes eutropha* or *Ralstonia eutropha*). The following simplified general reaction scheme describes aerobic $CO_2$ fixation via $H_2$ oxidation with $O_2$ as electron acceptor by Knallgas bacteria: $H_2 + O_2 + CO_2 \rightarrow$ biomass + $H_2O$.

The term "chemotrophic" relates to organisms using oxidation of chemical compounds as energy source, as opposed to "phototrophic" organisms, deriving their energy from light. The term "chemolithotrophic" relates to organisms using inorganic chemical compounds, for example hydrogen gas, as electron donor, in contrast to the term "chemoorganotrophic", which relates to organisms using organic compounds as electron donor. The terms "autotroph" or "heterotroph" relate to the carbon source, $CO_2$ being the carbon source for "autotrophic" organisms, organic carbon compounds being the carbon source for "heterotrophic" organisms. The term "mixotrophic" may be used for organisms being able to use both organic compounds and $CO_2$ as a carbon source. The term "chemolithoautotrophic" relates to organisms using inorganic compounds as energy source and electron donor, and $CO_2$ as a carbon source. The term "facultative chemolithoautotrophic" denotes organisms being able to also grow chemoorganoheterotrophically. "Knallgas" bacteria, for example, grow heterotrophically when Hz is at tropospheric concentrations and grow chemolithotrophically only when $H_2$ is available at higher concentrations (Pumphrey GM, Ranchou-Peyruse A, Spain JC., 2011, Cultivation- independent detection of autotrophic hydrogen-oxidizing bacteria by DNA stable-isotope probing, Appl Environ Microbiol. 77(14):4931-4938, doi:10.1128/AEM.00285-11).

The term "yeast" relates to eukaryotic unicellular fungal microorganisms. Examples of yeasts are *Saccharomyces cerevisiae*, *Candida albicans*, and *Yarrowia lipolytica.* The term "oleaginous yeast" relates to yeasts that are able to accumulate 20% and more of their cell dry-weight in the form of lipids (e.g. fats; see, for example, Abeln, E., Chuck, C.J., 2021, The history, state of the art and future prospects for oleaginous yeast research, Microb Cell Fact 20, 221 doi:10.1186/s12934-021-01712-1; Blomqvist, J., Pickova, J., Tilami, S.K. et al., 2018, Oleaginous yeast as a component in fish feed, Sci Rep 8, 15945, doi:10.1038/s41598-018- 34232-x). Examples of oleaginous yeasts are *Cutaneotrichosporon oleaginosus*, *Rhodotorula toruloides*, *Yarrowia lipolytica, Lipomyces starkeyi, Trichosporon oleaginosus* (formerly *Cryptococcus curvatus*) and *Rhodosporidium toruloides.*

The term "electrolyzing water" relates to a process in which direct electric current is used to split water into hydrogen and oxygen. The term "electrolyzer" is used to denote an apparatus being able to electrolyze water.

The term "hydrogen" relates, unless otherwise expressly stated or clearly evident from the context, to dihydrogen, i.e. the elemental molecule $H_2$ consisting of two hydrogen atoms joined by a single bond. The terms "molecular hydrogen", "molecular $H_2$", or "gaseous hydrogen" may also synonymously be used for Hz.

The term "oxygen" relates, unless otherwise expressly stated or clearly evident from the context, to dioxygen, i.e. the elemental molecule $O_2$ consisting of two oxygen atoms joined by a single bond. The terms "molecular oxygen", "molecular $O_2$", or "gaseous oxygen" may also synonymously be used for $O_2$.

The term "nitrogen" relates, unless otherwise expressly stated or clearly evident from the context, to dinitrogen, i.e. the elemental molecule $N_2$ consisting of two nitrogen atoms joined by a single bond. The terms "molecular nitrogen", "molecular $N_2$", or "gaseous nitrogen" may also synonymously be used for $N_2$.

The terms "C-source" or "carbon source" relate to chemical compounds containing at least one carbon atom that can be or is incorporated into chemical compounds constituting cellular material. An example of a C-source, in particular for autotrophic bacteria, is $CO_2$.

The terms "$CO_2$ fixation" or "carbon fixation" relate to the biological incorporation of inorganic carbon, in particular in the form of carbon dioxide, into organic carbon compounds, for example carbohydrates. The term "non-photosynthetic biological fixation of carbon dioxide" relates to carbon fixation not involving photosynthesis, i.e. by non-photosynthetic organisms. The terms "fermentation" or "fermentative" refer to any process in which the activity of microorganisms brings about a desirable change to a chemical compound, foodstuff or beverage.

The present inventors have dedicated themselves to solving the problem of the present invention and were successful to develop a novel and useful process for controlling gas fermentation, in particular oxyhydrogen fermentation, in order to lose as little gas as possible, compensate for concentration fluctuations and improve the overall balance. In addition, the inventors were successful in developing a bioreactor suitable for the process of the invention in which the gas input can be precisely adjusted as needed in order to improve the biomass and product formation.

Accordingly, the present invention provides a process for controlling gas fermentation. The process comprises a bioprocess comprising cultivation of a microorganism in a liquid medium in a bioreactor as a liquid culture. During the bioprocess, gases comprising molecular $H_2$ and $CO_2$ are introduced into the bioreactor and at least partially dissolved into the liquid culture, the partial pressure of each of the gases in a gaseous phase in a headspace above the liquid culture is measured, preferably continuously measured, and the volumetric flow rate of each of the introduced gases is continuously controlled such that the measured partial pressure of each of the gases in the bioreactor is controlled to a corresponding predetermined set partial pressure. Within the present invention, cultivation of a microorganism may be understood as cultivation of a single type of microorganisms or cultivation of two or more different microorganisms. In a preferred embodiment, cultivation of a microorganism means cultivation of a single type of microorganism. In another preferred embodiment, cultivation of a microorganism means cultivation of two different types of microorganism.

In a preferred embodiment of the process of the invention, the microorganism cultivated in the bioprocess is a bacterium, preferably a chemolithotrophic bacterium, more preferably a chemolithoautotrophic bacterium, even more preferably an oxyhydrogen bacterium, particularly preferably from the family *Hydrogenophilaceae*, particularly preferably of the species *Hydrogenophilus thermoluteolus*. However, the microorganism(s) is/are not limited to this and can be readily selected by the person skilled in the art depending on the intended application. For example, the microorganism cultivated in the bioprocess may also be *Hydrogenobacter thermophilus*, *Hydrogenovibrio marinus*, *Cupriavidus metallidurans*, *Rhodococcus opacus*, *Xantobacter autotrophicus*, and/or *Cupriavidus necator* (formerly *Alcaligenes eutropha* or *Ralstonia eutropha*). In a preferred embodiment of the process of the invention, a mixture of two or more different bacteria is cultivated in the bioprocess. In another particularly preferred embodiment of the process of the invention, the bioprocess is a gas-consuming process producing biomass.

In another preferred embodiment of the process of the invention, wherein at least 3 different gases, at least 4 different gases, or at least 5 different gases are introduced into the bioreactor during the bioprocess. Preferably, besides $H_2$ and $CO_2$, molecular $O_2$, molecular $N_2$, $CH_4$, and/or CO are introduced into the bioreactor during the bioprocess. The gases introduced may further comprise hydrocarbons $C_XH_Y$, nitrogen oxides NOx sulfur oxides SOx, and/or noble gases.

In another preferred embodiment of the process of the invention, any of the introduced gases is introduced separately, or at least two or more thereof are introduced together in form of a gas mixture, preferably wherein the gas mixture comprises $CO_2$, $O_2$, $N_2$, and/or $CH_4$. The gas mixtures may be, for example, flue gases captured in industrial process involving combustion or cement waste gas, or biogas. For example, depending on the combustion material and the management and completeness of the combustion, the gas phase of flue gases usually consists to a large extent of $N_2$, to a lesser extend of water vapor, $CO_2$, and $O_2$, and traces of CO, $C_XH_Y$, $NO_X$, $SO_X$, and/or noble gases.

In another preferred embodiment of the process of the invention, the total pressure during the bioprocess in the bioreactor is controlled to a predetermined set total pressure equal to or above atmospheric pressure, preferably above atmospheric pressure. Preferably, the set total pressure is between at least 1 bar to at most 10 bar, preferably between at least 1.5 bar to at most 6 bar, more preferably between 2 bar and 4 bar, particularly preferably at about 3 bar.

Increased pressure to a certain point enhances the solubility of gases in the culture medium, may improve mass transfer rates which results in more efficient nutrient and gas supply to the cells and more effective removal of waste products, and may reduce foam formation by minimizing gas bubble formation, which improves efficiency of the process, leading to higher product concentration or yield in a shorter time.

In another preferred embodiment of the process of the invention, the partial pressure of any of the gases in the gas atmosphere is controlled by a separate controller, preferably a PI, more preferably a PID controller, particularly preferably a mass flow controller driven by a PID logic, configured for controlling the volumetric flow rate of the corresponding introduced gas or a gas mixture comprising said corresponding introduced gas.

In another preferred embodiment of the process of the invention, the bioprocess is performed at a temperature between 20 to 60 °C, preferably between 30 to 58 °C, more preferably between 40 to 56 °C, particularly preferably between 45 to 54 °C, particularly preferably between 50 to 52 °C.

In another preferred embodiment of the process of the invention, at most 5.0, 4.0, 3.0, 2.0, 1.0, 0.9, 0.8, 0.7, 0.6, 0.5, 0.4, 0.3, 0.2, or 0.1 vol% of the $H_2$, $CO_2$, and/or $O_2$ present in the bioreactor is removed from the bioreactor during the bioprocess per hour. In an even more preferred embodiment of the process of the invention, at most 0.3, 0.2, or 0.1 vol%, particularly preferably at most 0.1 vol% of the $H_2$, $CO_2$, and/or $O_2$ present in the bioreactor is removed from the bioreactor during the bioprocess per hour. In a particularly preferred embodiment of the process of the invention, at most 0.1 vol%, particularly preferably at most 0.1 vol% of the $H_2$, $CO_2$, and/or $O_2$ present in the bioreactor is removed from the bioreactor during the bioprocess per hour.

In the case in which gas is removed from the bioreactor for the purpose of measuring the partial pressure(s) of $H_2$, $CO_2$, and/or $O_2$ in the headspace, the volume flow of the released gas must be at least 0.1 L/h, preferably at least 0.2 L/h, more preferably at least 0.3 L/h, even more preferably at least 0.4 L/h, even more preferably at least 0.5 L/h, particularly preferably at least 0.2 L/h 0.6 L/h.

In another preferred embodiment of the process of the invention, no $H_2$, $CO_2$, and/or $O_2$ present in the bioreactor is removed from the bioreactor. This may be the case in which the partial pressure measurement is carried out, for example, inline. The skilled person readily understands that the term "no $H_2$, $CO_2$, and/or $O_2$ is removed" also encompasses small gas leaks during the bioprocess as absolute gas tightness is hardly achievable in practice due to the multi-part structure of a bioreactor.

In another preferred embodiment of the process of the invention, excess $N_2$ and/or excess $CH_4$ is removed in a controlled manner from the bioreactor, preferably by membrane-based separation of $N_2$ and/or $CH_4$. The term "excess" in this context refers to the actual fraction of $N_2$ and/or $CH_4$ in the bioreactor that exceeds the target fraction of $N_2$ and/or $CH_4$ set in the bioprocess. For example, if the target fraction of $N_2$ is set at 80 vol% and the actual fraction of $N_2$ is 85 vol%, the bioreactor contains a 5 vol% excess of $N_2$ which is then removed from the bioreactor, or if the target fractions of $H_2$ are above 75 vol% and of $O_2$ above 10 vol%, the amount of $N_2$ and/or $CH_4$ that must be removed from the bioreactor in order to reach and maintain the target fractions of $H_2$ and $O_2$ is excess. If the target fraction of $N_2$ and/or $CH_4$ is set at 0 vol%, any amount of the gas(es) in the bioreactor is excess and is removed from the bioreactor.

In another preferred embodiment of the process of the invention, in cases wherein the gas atmosphere contains no $N_2$, the gas atmosphere in the bioreactor comprises 21 vol% or less of $O_2$, 15 vol% or less of $CO_2$, and more than 75 vol% of $H_2$, preferably about 76 vol% of $H_2$, about 14 vol% of $O_2$, and about 10 vol% of $CO_2$. Alternatively, in cases wherein the gas atmosphere contains $N_2$, the gas atmosphere in the bioreactor comprises 21 vol% or less of $O_2$, 15 vol% or less of $CO_2$, and less than 4 vol% of $H_2$, with the remaining fraction of the gas atmosphere being saturated by $N_2$. The person skilled in the art will readily understand that the term "no $N_2$" also encompasses that a small amount of $N_2$ may nevertheless be present in the bioreactor due to natural impurities of gases introduced into the bioreactor and unavoidable leakage during the bioprocess.

$H_2$ supplied into the bioreactor may be derived from a second bioprocess, a local source such as the $H_2$ side of an electrolyzer, or a reservoir such as a pressurized gas cylinder, and/or a pipeline. $CO_2$ and/or a gas mixture comprising $CO_2$, $O_2$, $N_2$, and/or $CH_4$ supplied into the bioreactor may be derived from a reservoir or pipeline, and the gas mixture may be industrial combustion gas, cement waste gas, and/or biogas from another bioprocess. By using waste gas mixtures containing $CO_2$ produced in an industrial process, overall $CO_2$ emissions as well as the costs of the bioprocess can be reduced. $O_2$ supplied into the bioreactor may be derived from a second bioprocess, a local source such as the $O_2$ side of an electrolyzer, a compressor, or a reservoir such as a pressurized gas cylinder, and/or a pipeline, and may be supplied in form of concentrated $O_2$ and/or compressed air.

In a particular preferred embodiment of the process of the invention, at least part of the $O_2$ is supplied into the bioreactor in form of compressed air. By introducing compressed air comprising about 21 vol% of $O_2$ into the bioreactor instead of concentrated $O_2$, the costs of the bioprocess can be further reduced.

In another preferred embodiment of the process of the invention, the liquid culture is agitated during the bioprocess by circulating gas from the gaseous phase in a headspace above the liquid culture into the liquid phase (gas cycling), by introducing gas from external sources into the liquid phase, by mixer agitation, and/or by circulating and spraying liquid culture from the liquid phase into the headspace (liquid cycling). Preferably, any of the agitation used also serves as a means of increasing gas input into the liquid culture.

Along with or instead of cycling the liquid culture from the liquid phase to the gas phase in a closed system, liquid cycling may also be performed by spraying liquid culture from an outside source such as a second bioreactor into the headspace, whereby the amount of liquid culture from the liquid phase from the bioreactor equal to the amount of external liquid culture introduced into the bioreactor should then be removed from the liquid phase so as not to affect the overall pressure. The removed liquid culture may then be transferred to the second bioreactor, for example.

According to another preferred embodiment of the process of the invention, the process is for controlling growth rate and/or production rate of the cultured microorganism.

The present invention further provides apparatuses configured to perform the processes for controlling gas fermentation according to the invention.

As such, the present invention further provides an apparatus 1 for cultivation of microorganisms. The apparatus 1 of the inventions comprises a bioreactor 2 configured for carrying out a bioprocess, wherein the bioprocess comprises the cultivation of microorganisms, preferably chemolithoautotrophic bacteria, in a liquid medium as a liquid culture 2a at a total pressure, preferably above atmospheric pressure, and wherein the bioprocess is a gas-consuming process producing biomass, preferably wherein the consumed gases comprise molecular hydrogen $H_2$, $CO_2$, and molecular oxygen $O_2$. The apparatus 1 of the invention further comprises a control unit comprising monitoring and analysis means 12 configured for measuring and analyzing process parameters during the bioprocess and a plurality of PI controllers, preferably PID controllers, particularly preferably mass flow controllers driven by a PID logic, wherein each controller is configured for controlling the volumetric flow rate of a gas introduced into the bioreactor based on data of the analysis means 12 as outlined by the process of the preceding claims, first gas separation means 9, preferably a separation membrane, configured for removing $N_2$ and/or $CH_4$ in a controlled manner from the bioreactor 2, a first fluid connection 4a fluidically connecting an outlet 4b of a first fluid connection 4a to a first inlet 4c of the bioreactor 2 and configured for the transfer of a first gas, preferably $H_2$, from the first external gas source 4 into the bioreactor 2, a second fluid connection 5a fluidically connecting an outlet 5b of a second external gas source 5 to a second inlet 5c of the bioreactor 2 and configured for the transfer of a second gas, preferably $CO_2$ and/or a gas mixture comprising $CO_2$, $O_2$, $N_2$, and/or $CH_4$, from the second external gas source 5 into the bioreactor 2, and a third fluid connection 6a fluidically connecting an outlet 6b of a third external gas source 6 to a third inlet 6c of the bioreactor 2, configured for the transfer of a third gas, preferably $O_2$, from the third external gas source 6 into the bioreactor 2.

In another preferred embodiment of the apparatus of the invention, the chemolithoautotrophic bacterium cultivated in the bioprocess is an oxyhydrogen bacterium, preferably from the family *Hydrogenophilaceae,* particularly preferably of the species *Hydrogenophilus thermoluteolus.* However, the microorganism(s) is/are not limited to this and can be readily selected by the person skilled in the art depending on the intended application. For example, the microorganism cultivated in the bioprocess may also be *Hydrogenobacter thermophilus*, *Hydrogenovibrio marinus*, *Cupriavidus metallidurans*, *Rhodococcus opacus*, *Xantobacter autotrophicus*, or *Cupriavidus necator* (formerly *Alcaligenes eutropha* or *Ralstonia eutropha*). In a preferred embodiment of the apparatus 1 of the invention, a mixture of two or more different bacteria is cultivated in the bioprocess. In another preferred embodiment of the apparatus 1 of the invention, the bioprocess is a gas-consuming process producing biomass.

In another preferred embodiment of the apparatus 1 of the invention, the apparatus 1 further comprises an electrolyzer 3 configured for the electrolysis of water into $O_2$ and $H_2$ and comprising a separator configured for separating $O_2$ and $H_2$, wherein the $H_2$ side of the separator is the first external gas source 4 and the $O_2$ side of the separator is the third external gas source 5, a fourth fluid connection 7a fluidically connecting a first outlet 7b of the bioreactor 2 located at liquid culture level to a fourth inlet 7c of the bioreactor 2 located at headspace level and configured for circulation of liquid culture to the headspace 2b, preferably wherein the fourth inlet 7c comprises spraying means 7 configured for spraying the circulated liquid culture in the headspace, a fifth fluid connection 8a fluidically connecting a second outlet 8b of the bioreactor 2 located at headspace level to a fifth inlet 8c of the bioreactor 2 located at liquid culture level and configured for circulation of the gas from the headspace 2b into the liquid culture 2a, preferably wherein the fifth inlet 8c comprise sparger means 8 configured for dispersing the circulated gas in the liquid culture 2a, a sixth fluid connection 9a fluidically connected to the fifth fluid connection 8a and comprising the first gas separation means 9, and/or a second gas separation means 13, preferably a gas separation membrane, provided in the third fluid connection 6a and configured for the removal of $N_2$ and/or $CH_4$ from the second gas transferred through the third fluid connection 6a into the bioreactor 2. The second gas separation means 13 is for complete or partial removal of $N_2$ and/or $CH_4$ in the second gas in the case in which at least part of the second gas is a gas mixture comprising $CO_2$, $O_2$, $N_2$, and/or $CH_4$. The first separation means 9 is for complete or partial removal of $N_2$ and/or $CH_4$ from the bioreactor 2, particularly in the case in which the gases accumulate during the bioprocess.

Within the present invention, "located at liquid culture level" refers to a positon below the surface of the liquid culture 2a, and "at headspace level" refers to a position above the surface of the liquid culture 2a. For example, as shown in Fig. 1, the first outlet 7b may be located at any position on the bioreactor that is below the surface of the liquid culture 2a, and the fourth inlet 7c may be located at any position on the bioreactor that is above the surface of the liquid culture 2a.

In another preferred embodiment of the apparatus 1 of the invention, in each of the fifth fluid connection 8a and the sixth fluid connection 9a a valve 9b, 9c is provided, configured to adjust a gas flow through the fifth fluid connection 8a and the sixth fluid connection 9a, respectively. The valves 9b, 9c allow the adjustment of the volume flows of the gas transferred from the head space 2b passing through the sixth fluid connection 9a via the first gas separation means 9 and/or through the fifth fluid connection 8a, bypassing the first gas separation means 9.

In another preferred embodiment of the apparatus 1 of the invention, the first inlet 4c, second inlet 5c, and third inlet 6c are located at liquid culture level, preferably at the bottom of the bioreactor 2, and comprise sparger means 10 configured for dispersing gas in the liquid culture 2a.

In a preferred embodiment of apparatus 1 of the invention, the first gas is Hz, the second gas is $CO_2$ and/or a gas mixture comprising $CO_2$, $O_2$, $N_2$, and/or $CH_4$, and the third gas is $O_2$. The first external gas source 4 supplying the first gas Hz into the bioreactor 2 may be a second bioprocess, a local source such as the $H_2$ side of the electrolyzer 3 or a reservoir such as a pressurized gas cylinder, and/or a pipeline. The second external gas source 5 supplying the second gas $CO_2$ and/or a gas mixture comprising $CO_2$, $O_2$, $N_2$, and/or $CH_4$ into the bioreactor 2 may be a reservoir or pipeline, and the gas mixture comprising $CO_2$, $O_2$, $N_2$, and/or $CH_4$ transferred via the pipeline or stored in the reservoir may be industrial combustion gas, cement waste gas, and/or biogas from another bioprocess. By using waste gas mixtures containing $CO_2$ produced in the industrial process, overall $CO_2$ emissions can be reduced, and the costs of the bioprocess can be reduced. The third external gas source 5 supplying the third gas $O_2$ into the bioreactor 2 may be a second bioprocess, a local source such as the $O_2$ side of the electrolyzer 3, a compressor, or a reservoir such as a pressurized gas cylinder, and/or a pipeline, and the third gas $O_2$ may be supplied in form of concentrated $O_2$ and/or compressed air.

In a particular preferred embodiment of the apparatus 1 of the invention, at least part of the $O_2$ is supplied into the bioreactor 2 in form of compressed air. By introducing compressed air comprising about 21 vol% of $O_2$ into the bioreactor instead of concentrated $O_2$, the costs of the bioprocess can be further reduced. All embodiments of the present invention as described herein are deemed to be combinable in any combination unless the skilled person considers such a combination to not make any technical sense.

## Example

An apparatus 1 according to the invention and modes for carrying out the method according to the invention are outlined below. The examples therein are for illustrative purposes only and are not intended to limit the scope of the invention.

As shown in an exemplary apparatus 1 according to the invention in Fig. 1, the gas fermentation process using a microorganism, for example *Hydrogenophilus thermoluteolus,* in a liquid culture 2a is performed in a closed pressure-resistant vessel of a bioreactor 2. The liquid culture is mixed by agitation using agitation means 11, for example in Fig. 1, a stirrer. Agitation with the agitation means 11 may also serve as a means of increasing gas input. For further increasing the gas input in the liquid medium during the fermentation, gas cycling from the headspace 2b into the liquid culture 2a (cf. Fig. 1) and liquid cycling of the liquid culture 2a through a spraying means like a nozzle for spraying into the headspace 2b are used. Liquid cycling may also be carried out using another reactor to saturate $H_2$, for example, outside the main reactor, as outlined in WO 2023/147838.

In the case *Hydrogenophilus thermoluteolus* is used as the microorganism, total pressure ($p_{sys_{total}}$) in the vessel of the bioreactor 2 may be controlled to a maximum 4 bar above atmospheric pressure as the inventors did not observe a significant improvement in terms of biomass and product productivity above 4 bar with *Cupriavidus necator.* $H_2$ and part of the $O_2$ for use in the gas fermentation are generated from an electrolyzer. $CO_2$ and part of the $O_2$ for use in the gas fermentation are introduced as an individual gas mix ("substrate gas") with fractions of $CO_2$, $N_2$, $O_2$, and trace amounts of Ar from a $CO_2$-emitting industry.

The substrate gas can be, for example, combustion gas, cement waste gas or biogas. In general, exhaust gases of industrial processes are processed by membrane-based concentration and $N_2$ separation. Consequently, they usually consist of significantly higher $CO_2$ fractions than $O_2$ fractions. As such, the substrate gas has to be supplemented with pure $O_2$ from the electrolyzer in order to reach the desired $O_2$ concentration during the bioprocess.

Gas control in the reactor system is based on continuous flow and concentration control of the incoming and outgoing gases, wherein the pressure $p_{sys_{total}}$ in the system is continuously monitored and controlled. During the process, target fractions of the gases $H_2$, $O_2$ and $CO_2$ are set at a predetermined pressure. For example, the target fractions in the gas mixture may be set to 76 vol% $H_2$, 14 vol% $O_2$ and 10 vol% $CO_2$ and the pressure $p_{sys_{total}}$ to 3 bar.

In order to pressurize the bioreactor 2 before the start of the process, $H_2$, $O_2$ and substrate gas were introduced into the bioreactor 2 at respective volume flows $q_{H_2}$, $q_{CO_2}$, and $q_{O_2}$. The volume flows $q_{H_2}$, $q_{CO_2}$, and $q_{O_2}$ in volume per time of the gases $H_2$, $O_2$ and substrate gas are calculated as described according to Equations E.1-E.3.

$$q_{H_2} = c_{set_{H_2}} * q_{set_{total}} \qquad\qquad E.1$$

$$q_{sub} = \frac{c_{set_{CO_2}}}{c_{sub_{CO_2}}} * q_{set_{total}} \qquad\qquad \text{E.2}$$

$$q_{O_2} = q_{set_{total}} - (q_{H_2} + q_{sub}) \qquad\qquad \text{E.3}$$

$$\frac{c_{sub_{CO_2}}}{c_{sub_{O_2}}} * \frac{c_{set_{O_2}}}{c_{set_{CO_2}}} \geq 1 \qquad\qquad \text{E.4}$$

$$q_{set_{total}} = q_{H_2} + q_{sub} + q_{O_2} \qquad\qquad \text{E.5}$$

With $c_{set_i}$ in vol% as the target fractions of the gases, $q_{set_{total}}$ as the desired total volume flow and $c_{sub_i}$ as the gas fractions in the substrate gas. Here, E.4 must be fulfilled or otherwise pure $CO_2$ must be mixed in using the same process. With E.5, the reactor system is brought to the desired total pressure $p_{set_{total}}$, whereby initially the gas to be displaced (e.g. $N_2$) is displaced and then no gas ($q_{ex}$, Fig. 1) escapes from the system via the exhaust line to build up pressure. The pressure in the system is regulated via a pressure gauge in conjunction with mass flow controllers for the respective gases.

The process is configured to supply only as much gas after the introduction of the microorganism as was actually consumed by the microorganism. As the microorganism has different gas uptake rates for $H_2$, $O_2$ and $CO_2$ depending on the species, growth phase and product, the partial pressures in the system will shift during the bioprocess. As such, in order to determine the actual gas fractions $c_{sys_i}$ of $H_2$, $O_2$ and $CO_2$ in the bioreactor 2, a low exhaust gas flow $q_{ex}$ (~10 mL/min) is let out of the system in order to be analyzed by analysis means 12 (e.g. mass spectrometer).

In the course of the bioprocess, if no gas is readjusted, total pressure $p_{set_{total}}$ in the system would decrease due to gas consumption of the microorganisms. Therefore, the entire gas inflow is upregulated to maintain the pressure. Consequently, the partial gas flows $q_{H_2}$, $q_{CO_2}$, and $q_{O_2}$ contained therein are upregulated based on the consumed fractions, known from $c_{sys_i}$. To maintain the total pressure $p_{set_{total}}$ in the system, partial pressures $p_{sys_i}$ are used. For this purpose, the partial pressures $p_{sys_i}$ are preferably controlled separately, e.g. one partial pressure $p_{sys_i}$ with one PI controller each. As the partial pressures $p_{sys_i}$ are interdependent via the total pressure $p_{sys_{total}}$ in the system (E.6), undesired build-up of the pressure is prevented. However, since the partial pressures $p_{sys_i}$ refer to the pure gases and $q_{sub}$ of $CO_2$ and $O_2$ comprises, $p_{set_i}(t)$ must be adjusted analogously to E.1-3 (E.7-E.9).

$$p_{sys_i}(t) = c_{sys_i}(t) * p_{sys_{total}}(t) \qquad\qquad \text{E.6}$$

$$p_{set_{H_2}}(t) = c_{set_{H_2}}(t) * p_{set_{total}}(t) \qquad\qquad \text{E.7}$$

$$p_{set_{sub}}(t) = \frac{c_{set_{CO_2}}(t)}{c_{sub_{CO_2}}(t)} * p_{set_{total}}(t) \qquad\qquad \text{E.8}$$

$$p_{set_{O_2}}(t) = p_{set_{total}}(t) - \left(p_{set_{H_2}}(t) + p_{set_{sub}}(t)\right) \qquad \text{E.9}$$

$$e_i(t) = p_{set_i}(t) - p_{sys_i}(t) \qquad\qquad \text{E.10}$$

To control the partial pressures $p_{sys_i}$ over the fermentation time window t, a deviation variable $e_i(t)$ is formed from the difference between the partial pressures $p_{set_i}$ and $p_{sys_i}$ (setpoint and process value) of the gases (E.10). With a PI controller for each of the gas components, $c_{sys_i}$ can now be brought to approximate the target variables $c_{set_i}$ while maintaining the total pressure $p_{sys_{total}}$ as near as possible to $p_{set_{total}}$. In the controller, $u_i(t)$ is defined as the output value, so that a typical PI controller equation with the proportional factor $K_p$ and integration time $T_N$ is obtained (E.11). $u_i$ is then proportional to the applied volumetric flow $q_i$ (E.12) of the respective gas i depending on the specification of the mass flow controller.

$$u_i(t) = K_p * \left[ e_i(t) + \frac{1}{T_N} * \int_0^t e_i(\tau)d\tau \right] \quad \text{E.11}$$

$$u_i \propto q_i \qquad\qquad \text{E.12}$$

This controller is used for the corresponding feed streams $H_2$, $O_2$ and substrate gas. By integrating the substrate gas inflow into the gas analysis, a varying $CO_2$ or $O_2$ content in the substrate is compensated for in compliance with the condition from E.4.

In the case the fermentation takes place below the LEL of $H_2$ due to the specifications of the $CO_2$ emitter or the requirements of the bioprocess, $N_2$, for example, must be contained in the substrate gas or added as an inert gas. However, with the pressure-controlled regulation of the gassing, $N_2$ would accumulate as it is not consumed. In this case, $N_2$ separation means (e.g. membrane-based) are integrated into the gas cycling side. Utilizing upstream valves (a/b, Fig. 1), the flow rate at which the gas in the bioreactor 2 is passed over the $N_2$ separation membrane can be controlled. In this case, an additional PI controller may be used with the flow rate (depending on the valve position) of the gas via the separation membrane as a control variable and a reference variable analogous to E.10 with $N_2$.

**Reference signs**

| | |
|---|---|
| Apparatus | 1 |
| Bioreactor | 2 |
| Liquid culture | 2a |
| Headspace | 2b |
| Electrolyzer | 3 |
| First external gas source | 4 |
| First fluid connection | 4a |
| Outlet | 4b |
| First inlet | 4c |
| Second external gas source | 5 |
| Second fluid connection | 5a |
| Outlet | 5b |
| Second inlet | 5c |
| Third external gas source | 6 |
| Third fluid connection | 6a |
| Outlet | 6b |
| Third inlet | 6c |
| Spraying means | 7 |
| Fourth fluid connection | 7a |
| First outlet | 7b |
| Fourth inlet | 7c |
| Sparger means | 8 |
| Fifth fluid connection | 8a |
| Second outlet | 8b |
| Fifth inlet | 8c |
| First separation means | 9 |
| Sixth fluid connection | 9a |
| Valve | 9b |
| Valve | 9c |
| Sparger means | 10 |
| Agitation means | 11 |
| Analysis means | 12 |
| Second separation means | 13 |

**Claims**

1. A process for controlling gas fermentation, wherein the process comprises a bioprocess comprising cultivation of a microorganism in a liquid medium as a liquid culture in a bioreactor, wherein during the bioprocess,

   i. gases comprising molecular $H_2$ and $CO_2$ are introduced into the bioreactor and at least partially dissolved into the liquid culture;
   ii. the partial pressure of each of the gases in a gaseous phase in a headspace above the liquid culture is measured; and
   iii. the volumetric flow rate of each of the introduced gases is continuously controlled such that the measured partial pressure of each of the gases in the bioreactor is controlled to a corresponding predetermined set partial pressure.

2. The process according to claim 1, wherein the microorganism is a bacterium, preferably a chemolithotrophic bacterium, more preferably a chemolithoautotrophic bacterium, even more preferably an oxyhydrogen bacterium, particularly preferably from the family *Hydrogenophilaceae,* particularly preferably of the species *Hydrogenophilus thermoluteolus.*

3. The process according to claim 1 or 2, wherein at least 3 different gases, at least 4 different gases, or at least 5 different gases are introduced into the bioreactor during the bioprocess.

4. The process according to any of claim 1 to 3, wherein additionally molecular $O_2$, molecular $N_2$, $CH_4$ and/or CO are introduced into the bioreactor during the bioprocess, and/or wherein any of the introduced gases is introduced separately, or at least two or more thereof are introduced together in form of a gas mixture, preferably wherein the gas mixture comprises $CO_2$, $O_2$, $N_2$, and/or $CH_4$.

5. The process according to any one of the preceding claims, wherein the total pressure during the bioprocess in the bioreactor is controlled to a predetermined set total pressure equal to or above atmospheric pressure, preferably above atmospheric pressure.

6. The process according to claim 5, wherein the set total pressure is between at least 1 bar to at most 10 bar, preferably between at least 1.5 bar to at most 6 bar, more preferably between 2 bar and 4 bar, particularly preferably at about 3 bar.

7. The process according to any one of the preceding claims, wherein the partial pressure of any of the gases in the gas atmosphere is controlled by a separate controller, preferably a PI controller configured for controlling the volumetric flow rate of the corresponding introduced gas or a gas mixture comprising said corresponding introduced gas.

8. The process according to any one of the preceding claims, wherein the bioprocess is performed at a temperature between 20 to 60 °C, preferably between 30 to 58 °C, more preferably between 40 to 56 °C, particularly preferably between 45 to 54 °C, particularly preferably between 50 to 52 °C.

9. The process according to any one of claims 4 to 8, wherein less than 0.1 vol% of the $H_2$, $CO_2$, and/or $O_2$ present in the bioreactor is removed from the bioreactor during the bioprocess per hour; or wherein $H_2$, $CO_2$, and/or $O_2$ present in the bioreactor is not removed from the bioreactor during the bioprocess, and/or wherein excess $N_2$ and/or excess $CH_4$ is removed in a controlled manner from the bioreactor, preferably by membrane-based separation of $N_2$ and/or $CH_4$.

10. The process according to any one of claims 4 to 9, wherein the gas atmosphere in the bioreactor comprises 21 vol% or less of $O_2$, 15 vol% or less of $CO_2$, and

    a. more than 75 vol% of $H_2$, in cases wherein the gas atmosphere contains no $N_2$, preferably about 76 vol% of $H_2$, about 14 vol% of $O_2$, and about 10 vol% of $CO_2$; or
    b. less than 4 vol% of $H_2$, in cases wherein the gas atmosphere contains $N_2$.

11. The process according to any one of the preceding claims, wherein the liquid culture is agitated during the bioprocess by circulating gas from the gaseous phase in a headspace above the liquid culture into the liquid phase (gas cycling), by introducing gas from external sources into the liquid phase, by mixer agitation, and/or by circulating and spraying liquid culture from the liquid phase into the headspace (liquid cycling).

12. The process according to any one of the preceding claims, wherein the process is for controlling growth rate and/or production rate of the cultured microorganism.

13. An apparatus (1) for cultivation of microorganisms, the apparatus (1) comprising:

    i. a bioreactor (2) configured for carrying out a bioprocess, wherein the bioprocess comprises the cultivation of microorganisms, preferably chemolithoautotrophic bacteria, in a liquid medium as a liquid culture (2a) at a total pressure, preferably above atmospheric pressure, and wherein the bioprocess is a gas-consuming process producing biomass, preferably wherein the consumed gases comprise molecular oxygen $O_2$, molecular hydrogen $H_2$, and $CO_2$;

    ii. a control unit comprising monitoring and analysis means (12) configured for measuring and analyzing process parameters during the bioprocess and a plurality of PI controllers, wherein each PI controller is configured for controlling the volumetric flow rate of a gas introduced into the bioreactor based on data of the analysis means (12) as outlined by the process of the preceding claims;

    iii. first gas separation means, preferably a separation membrane, configured for removing $N_2$ and/or $CH_4$ in a controlled manner from the bioreactor;

    iv. a first fluid connection (4a) fluidically connecting an outlet (4b) of a first external gas source (4) to a first inlet (4c) of the bioreactor (2) and configured for the transfer of a first gas, preferably $H_2$, from the first external gas source (4) into the bioreactor (2);

    v. a second fluid connection (5a) fluidically connecting an outlet (5b) of a second external gas source (5) to a second inlet (5c) of the bioreactor (2) and configured for the transfer of a second gas, preferably $CO_2$ and/or a gas mixture comprising $CO_2$, $O_2$, $N_2$, and/or $CH_4$, from the second external gas source (5) into the bioreactor (2); and

    vi. a third fluid connection (6a) fluidically connecting an outlet (6b) of a third external gas source (6) to a third inlet (6c) of the bioreactor (2), configured for the transfer of a third gas, preferably $O_2$, from the third external gas source (6) into the bioreactor (2).

14. The apparatus according to claim 13, further comprising:

    i. an electrolyzer (3) configured for the electrolysis of water into $O_2$ and $H_2$ and comprising a separator configured for separating $O_2$ and $H_2$, wherein the $H_2$ side of the separator is the first external gas source (4) and the $O_2$ side of the separator is the third external gas source (5);

    ii. a fourth fluid connection (7a) fluidically connecting a first outlet (7b) of the bioreactor (2) located at liquid culture level to a fourth inlet (7c) of the bioreactor (2) located at headspace level and configured for circulation of liquid culture (2a) to the headspace (2b), preferably wherein the fourth inlet (7c) comprises spraying means (7) configured for spraying the circulated liquid culture (2a) in the headspace;

    iii. a fifth fluid connection (8a) fluidically connecting a second outlet (8b) of the bioreactor (2) located at headspace level to a fifth inlet (8c) of the bioreactor (2) located at liquid culture level and configured for circulation of the gas from the headspace (2b) into the liquid culture (2a), preferably wherein the fifth inlet (8c) comprise sparger means (8) configured for dispersing the circulated gas in the liquid culture (2a);

    iv. a sixth fluid connection (9a) fluidically connected to the fifth fluid connection (8a) and comprising the first gas separation means (9); and/or

    v. a second gas separation means (13), preferably a gas separation membrane, provided in the third fluid connection (6a) and configured for the removal of $N_2$ and/or $CH_4$ from the second gas transferred through the third fluid connection (6a) into the bioreactor 2.

15. The apparatus according to claim 13 or 14, wherein the first inlet (4c), second inlet (5c), and third inlet (6c) are located at liquid culture level, preferably at the bottom of the bioreactor (2), and comprise sparger means (10) configured for dispersing gas in the liquid culture (2a).

**FIG. 1**

Europäisches
Patentamt

European
Patent Office

Office européen
des brevets

# EUROPEAN SEARCH REPORT

**Application Number**

EP 24 16 4663

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2019/300838 A1 (SMITH GARY J [GB] ET AL) 3 October 2019 (2019-10-03) * the whole document * | 1-6 | INV. C12M1/00 C12M1/34 |
| X | AU 2018 214 626 A1 (KIVERDI INC [US]) 22 August 2019 (2019-08-22) * paragraph [0051] * * paragraph [0100] * * paragraph [0329] * * paragraph [0444] * * claims * * figures * | 1-6 | |
| X | US 2013/189763 A1 (DALLA-BETTA PETER [US] ET AL) 25 July 2013 (2013-07-25) * paragraph [0001] - paragraph [0006] * * paragraph [0010] * * paragraph [0035] * * paragraph [0043] - paragraph [0045] * * paragraph [0108] * * paragraph [0177] * * paragraph [0183] - paragraph [0191] * | 1-6 | |
| X | EP 2 376 616 B1 (UNIBIO AS [DK]) 8 January 2020 (2020-01-08) * paragraph [0028] * * paragraph [0060] * * paragraph [0077] * * paragraph [0080] * * paragraph [0086] * * claims * | 1-6 | TECHNICAL FIELDS SEARCHED (IPC) C12M |

The present search report has been drawn up for all claims

| Place of search | Date of completion of the search | Examiner |
|---|---|---|
| Berlin | 25 February 2025 | Panzica, Gian Paolo |

EPO FORM 1503 03.82 (P04C01)

## ANNEX TO THE EUROPEAN SEARCH REPORT
## ON EUROPEAN PATENT APPLICATION NO.

EP 24 16 4663

This annex lists the patent family members relating to the patent documents cited in the above-mentioned European search report.
The members are as contained in the European Patent Office EDP file on
The European Patent Office is in no way liable for these particulars which are merely given for the purpose of information.

25-02-2025

| Patent document cited in search report | Publication date | Patent family member(s) | | Publication date |
|---|---|---|---|---|
| US 2019300838 A1 | 03-10-2019 | CN | 111836898 A | 27-10-2020 |
| | | EP | 3775240 A1 | 17-02-2021 |
| | | US | 2019300838 A1 | 03-10-2019 |
| | | WO | 2019191761 A1 | 03-10-2019 |
| AU 2018214626 A1 | 22-08-2019 | AU | 2018214626 A1 | 22-08-2019 |
| | | AU | 2024201994 A1 | 18-04-2024 |
| | | BR | 112019015945 A2 | 24-03-2020 |
| | | CA | 3051478 A1 | 09-08-2018 |
| | | CL | 2019002160 A1 | 29-05-2020 |
| | | CL | 2022000064 A1 | 28-10-2022 |
| | | CL | 2024001858 A1 | 29-11-2024 |
| | | CN | 110678539 A | 10-01-2020 |
| | | EA | 201891926 A1 | 30-04-2019 |
| | | EA | 201991813 A1 | 29-01-2020 |
| | | EP | 3638766 A1 | 22-04-2020 |
| | | IL | 268158 A | 26-09-2019 |
| | | IL | 313961 A | 01-08-2024 |
| | | JP | 2020506708 A | 05-03-2020 |
| | | JP | 2024028821 A | 05-03-2024 |
| | | KR | 20190115038 A | 10-10-2019 |
| | | PH | 12019501781 A1 | 05-10-2020 |
| | | US | 2019390158 A1 | 26-12-2019 |
| | | US | 2020216797 A1 | 09-07-2020 |
| | | US | 2023122678 A1 | 20-04-2023 |
| | | US | 2025051714 A1 | 13-02-2025 |
| | | US | 2025059498 A1 | 20-02-2025 |
| | | WO | 2018144965 A1 | 09-08-2018 |
| US 2013189763 A1 | 25-07-2013 | US | 2013189763 A1 | 25-07-2013 |
| | | US | 2016102287 A1 | 14-04-2016 |
| | | WO | 2013090769 A2 | 20-06-2013 |
| EP 2376616 B1 | 08-01-2020 | BR | PI0923056 A2 | 11-08-2015 |
| | | CA | 2746696 A1 | 24-06-2010 |
| | | DK | 2376616 T3 | 06-04-2020 |
| | | EP | 2376616 A2 | 19-10-2011 |
| | | US | 2011244543 A1 | 06-10-2011 |
| | | WO | 2010069313 A2 | 24-06-2010 |

EPO FORM P0459

For more details about this annex : see Official Journal of the European Patent Office, No. 12/82

# EP 4 621 039 A1

## REFERENCES CITED IN THE DESCRIPTION

### Patent documents cited in the description

- WO 2011088364 A9
- WO 2009133351 A2
- WO 2011139804 A2
- WO 2023147838 A

### Non-patent literature cited in the description

- **BRIGHAM, C.** Perspectives for the biotechnological production of biofuels from CO2 and H2 using Ralstonia eutropha and other 'Knallgas' bacteria. *Applied Microbiology and Biotechnology*, 2019, vol. 103, 10
- **R. SANDER.** Compilation of Henry's law constants (version 4.0) for.. *Atmos. Chem. phys.*, 2015, 4399-4981
- Concise Dictionary of Biomedicine and Molecular Biology. CRC Press, 2002
- The Dictionary of Cell and Molecular Biology. Academic Press
- Oxford Dictionary Of Biochemistry And Molecular Biology, Revised. Oxford University Press, 2000
- **PUMPHREY GM** ; **RANCHOU-PEYRUSE A** ; **SPAIN JC.** Cultivation- independent detection of autotrophic hydrogen-oxidizing bacteria by DNA stable-isotope probing. *Appl Environ Microbiol.*, 2011, vol. 77 (14), 4931-4938
- **ABELN, E.** ; **CHUCK, C.J.** The history, state of the art and future prospects for oleaginous yeast research. *Microb Cell Fact*, 2021, vol. 20, 221
- **BLOMQVIST, J.** ; **PICKOVA, J.** ; **TILAMI, S.K. et al.** Oleaginous yeast as a component in fish feed. *Sci Rep*, 2018, vol. 8, 15945